# FASCICULE DE BREVET EUROPEEN

(11) **EP 2 601 607 B1**
(45) Date de publication et mention de la délivrance du brevet: **04.04.2018**
(21) Numéro de dépôt: 11754895.8
(22) Date de dépôt: 20.07.2011
(51) Int. Cl.: G06F 19/00

(54) **PROCEDE D'ACQUISITION AUTOMATISE ET ASSISTE DE SURFACES ANATOMIQUES**
VERFAHREN ZUR AUTOMATISIERTEN UND UNTERSTÜTZTEN ERFASSUNG ANATOMISCHER OBERFLÄCHEN
METHOD FOR THE AUTOMATED AND ASSISTED ACQUISITION OF ANATOMICAL SURFACES

(30) Priorité: 04.08.2010 FR 1056428
(43) Date de publication de la demande: 12.06.2013
(73) Titulaire: Medtech, 34170 Castelnau le Lez (FR)
(72) Inventeur: NAHUM, Bertin, F-34670 Baillargues (FR); BADANO, Fernand, F-69100 Villeurbanne (FR); MAILLET, Pierre, F-34130 Saint-Aunes (FR); HABERMEIER, Alexander, 1071 Amsterdam (NL); DEHOUR, Patrick, F-30260 Crespian (FR)
(74) Mandataire: Mays, Julie
(86) Numéro de dépôt international: PCT/FR2011/051747
(87) Numéro de publication internationale: WO 2012/017167

(56) Documents cités:
- FR-A1- 2 871 363
- FR-A1- 2 917 598
- TROCCAZ, J.: "Capteurs et recalage per-opératoires", , 1 décembre 2008 (2008-12-01), pages 1-32, XP002632288, Extrait de l'Internet: URL:http://cel.archives-ouvertes.fr/docs/0 0/34/47/21/PDF/Recalage-per-op-robots-medi caux.pdf [extrait le 2011-04-08]

## Description

La présente invention entre dans le domaine médical, en particulier dans la méthodologie opératoire lors de la préparation et la réalisation d'interventions chirurgicales.

L'invention concerne plus particulièrement l'imagerie médicale et, en phase per opératoire, l'acquisition automatisée de surfaces anatomiques, en particulier de la tête et du visage d'un patient, puis le recalage surfacique des images acquises par rapport à des images enregistrées en phase préopératoire.

L'invention trouvera une application dans l'aide assistée par robotique pour l'acquisition de surfaces anatomiques et pour le recalage surfacique.

Pour ce faire, la présente invention a pour objet un procédé d'acquisition automatisée et assistée de surfaces anatomiques.

De manière connue, lors d'une opération chirurgicale sur un patient, en particulier dans le cadre de neurochirurgie au niveau de la tête d'un patient, le médecin a recourt à des systèmes offrant une assistance, en particulier en améliorant la précision chirurgicale. Pour ce faire, de tels systèmes permettent, en phase per opératoire, l'acquisition des surfaces anatomiques visées par l'opération, puis leur recalage par rapport à des images déjà enregistrées, par exemple auparavant lors d'une phase préopératoire au cours d'un examen radiologique (CT-scan) ou bien d'un IRM (pour Imagerie par Résonance Magnétique). Ainsi, il est possible pour le médecin de localiser précisément le patient par rapport à l'imagerie en vue de l'opération.

Plus particulièrement, l'acquisition consiste à repérer la position réelle de la zone anatomique du patient, en effectuant un balayage de la surface de ladite zone à l'aide d'un pointeur, par exemple sous forme d'une pointe mécanique, d'ondes ultrason ou d'un faisceau laser. Le système effectue alors un recalage surfacique sous forme d'une comparaison entre ce repérage et les images enregistrées au préalable en phase préopératoire, calculant la mise en correspondance des images existantes avec le corps du patient au moment de l'opération. En somme, pour chaque point repéré, une évaluation est effectuée de manière à faire correspondre le nuage de points acquis avec les images préenregistrées.

Dès lors, la façon dont est réalisée l'étape d'acquisition des surfaces anatomiques, influence considérablement la précision de l'opération qui suivra. Plusieurs systèmes d'acquisition existent à ce jour, utilisant différentes techniques de repérage des surfaces anatomiques.

Une première solution consiste à positionner, en différents endroits particuliers de la zone à acquérir, des marqueurs, sous forme d'un masque ou de pastilles, collés directement sur la peau. Ces marqueurs sont ensuite repérés dans l'espace par balayage d'une pointe mécanique ou d'un faisceau d'émission/réception, notamment un laser.

L'inconvénient majeur d'un tel balayage réside dans son manque de précision, dépendant de la manière de localiser lesdits marqueurs ainsi que leur nombre et leur répartition spatiale sur la peau. Le recalage en découlant est alors peu fiable, à savoir qu'il présente des variations et des décalages importants au niveau des surfaces situées entre les marqueurs.

De plus, les marqueurs peuvent bouger, en raison de l'élasticité de la peau, voire se décoller. La pose des marqueurs oblige aussi à raser la partie du crâne.

Une solution alternative consiste à parcourir la zone anatomique à l'aide d'un pointeur dont les coordonnées sont situées dans l'espace, en particulier au travers de caméras.

Selon un mode de réalisation, ledit pointeur peut être mécanique, se présentant sous la forme d'un palpeur dont la pointe vient directement au contact de la peau. Ladite pointe est déplacée manuellement de point en point, notamment sur les points morphologiquement remarquables, et le long de lignes anatomiques particulières de la zone concernée, tandis que ses différentes positions et points de contact sont enregistrés en trois dimensions.

Toutefois, si cette technique permet de repérer un plus grand nombre de points de la surface, elle reste limitée dans le nombre de points repérés, de l'ordre d'une centaine, nécessitant une restriction du repérage à certaines lignes et certains endroits remarquables de l'anatomie du patient. Cette restriction, due à l'intervention de l'opérateur, influe automatiquement sur la qualité du recalage surfacique ultérieur. Par ailleurs, la déformation de la peau lors du balayage avec le pointeur est une autre cause d'imprécision.

Une alternative réside dans un pointeur sans contact, permettant d'obtenir un plus grand nombre de points repérés en un laps de temps moindre. Un tel pointeur se présente sous la forme d'un émetteur de rayonnement lumineux, tel un faisceau laser. Ledit émetteur est tenu manuellement par le praticien qui balaye la zone anatomique avec le laser.

Un premier dispositif connu comprend un émetteur sous forme d'un télémètre laser dont la position et l'orientation sont constamment repérées dans l'espace, permettant d'obtenir les coordonnées de chaque point enregistré par le télémètre.

Toutefois, la précision du repérage du télémètre reste limitée. C'est pourquoi il a été imaginé d'enregistrer directement l'impact au niveau de la peau du faisceau laser émis. Pour ce faire, l'émetteur émet, d'une part, un faisceau laser dans le spectre de lumière visible, pour permettre au praticien de visualiser le point d'impact et son balayage de la zone anatomique du patient et, d'autre part, un faisceau en lumière invisible, tels les infrarouges, qui sont captés par des capteurs spécifiques. Plus précisément, la réflexion des infrarouges au point d'impact permet de repérer précisément la position dudit point dans l'espace.

On notera que la localisation du télémètre ou du point d'impact du faisceau laser utilise un principe de triangulation optique à l'aide de plusieurs caméras.

Malgré ces différentes évolutions, les systèmes de repérage et de balayage existants n'apportent pas entière satisfaction. En effet, le balayage s'effectue toujours manuellement, créant un facteur humain qui diminue la précision du repérage, mais aussi son caractère répétable, à savoir que les trajectoires de balayage restent approximatives et entièrement liées au praticien.

Pour pallier ces inconvénients, il a été imaginé de coupler l'émetteur à un robot. De telles solutions sont décrites dans les documents WO 2009/013406, WO 2005/122916 et WO 2005/032390.

En particulier, l'émetteur est fixé à l'extrémité d'un bras robotisé, articulé de manière à présenter des degrés de liberté de mouvement dans les trois dimensions. La position de l'émetteur et des données qu'il enregistre, sont alors repérées dans l'espace par rapport au référentiel dudit bras robotisé.

Plus particulièrement, est effectuée une première acquisition préalable desdites surfaces anatomiques de manière à créer une représentation en trois dimensions sous forme d'un premier modèle numérique ; puis on effectue une seconde acquisition per opératoire par balayage desdites surfaces de manière à créer une représentation en trois dimensions sous forme d'un second modèle numérique ; puis ledit balayage étant réalisé à l'aide de moyens de repérage des coordonnées desdites surfaces, lesdits moyens étant supportés par un bras robotisé ; et enfin on effectue une mise en correspondance par recalage desdits premier et second modèles.

Dès lors, on constate que le recalage des modèles n'est pas optimal, nécessitant l'intervention d'un opérateur informatique, pour tenter de faire correspondre les modèles entre eux. En cas d'échec, il est alors nécessaire de répéter l'opération de balayage, augmentant d'autant la durée de l'intervention.

De plus, même si de tels dispositifs permettent de s'affranchir de la dépendance de l'opérateur, en automatisant le balayage de la surface anatomique, avec un caractère fortement reproductible, cette automatisation limite considérablement les capacités d'adaptation de ces dispositifs par rapport à la zone anatomique, en particulier par rapport aux différentes morphologies des patients.

Par ailleurs, dans tous les cas, les dispositifs existants utilisent des moyens de navigation au sein de la visualisation du modèle numérique en trois dimensions obtenu à partir des images ainsi acquises. Ces moyens de navigation nécessitent forcément le repérage de l'émetteur, comme précédemment évoqué, puis par la suite des instruments chirurgicaux.

L'invention a pour but de pallier les inconvénients de l'état de la technique en proposant un procédé d'acquisition automatisée et assistée de surfaces anatomiques qui combine la rigueur d'un balayage assisté par un bras robotisé avec l'adaptabilité d'un balayage manuel, tout en autorisant l'acquisition d'un grand nombre de points.

En particulier, l'invention prévoit d'effectuer un balayage préliminaire piloté manuellement par le praticien, avec un émetteur supporté par ledit bras robotisé, permettant, d'une part, de déterminer une zone spécifique pour un balayage ultérieur entièrement automatique et, d'autre part, d'effectuer un premier recalage surfacique augmentant la précision du recalage surfacique final.

Pour ce faire, dans un tel procédé :
- on effectue une première acquisition préalable desdites surfaces anatomiques de manière à créer une représentation en trois dimensions sous forme d'un premier modèle numérique ;
- on effectue une seconde acquisition per opératoire par balayage desdites surfaces de manière à créer une représentation en trois dimensions sous forme d'un second modèle numérique ;
ledit balayage étant réalisé à l'aide de moyens de repérage des coordonnées desdites surfaces, lesdits moyens étant supportés par un bras robotisé ;
puis
- on effectue une mise en correspondance par recalage desdits premier et second modèles.

Ledit procédé se caractérise en ce que ledit balayage consiste à :
- effectuer un repérage préliminaire des coordonnées de points remarquables desdites surfaces anatomiques par déplacement manuel, assisté par ledit bras robotisé, desdits moyens de repérage au niveau desdits points remarquables, de manière à construire un repère et à déterminer une zone de balayage desdites surfaces anatomiques ;
- créer un modèle intermédiaire à partir dudit repère et d'au moins un desdits points remarquables ;
- effectuer un recalage préliminaire dudit premier modèle avec ledit second modèle ;
- effectuer un balayage automatique de la zone déterminée.

Ainsi, le procédé selon l'invention offre une précision accrue dans les balayages effectués et le repérage d'une quantité plus importante de points anatomiques de la zone concernée, avec une précision automatisée et reproductible de la trajectoire, tout en ajoutant un caractère manuelle et adaptable au travers de la manipulation initiale par un opérateur.

Un autre avantage de la présente invention réside dans l'utilisation d'un bras robotisé qui sert alors de repère de référence. La zone anatomique à balayer puis le modèle extrait de cette acquisition sont localisés par rapport à ce repère de référence, de sorte que, par la suite, après recalage, le même repère de référence du bras robotisé sert à positionner les instruments chirurgicaux en vue de l'opération.

Selon d'autres caractéristiques, ledit procédé consiste à :
- effectuer un repérage des coordonnées d'au moins trois points remarquables ;
- déterminer un quatrième point à partir d'un desdits trois points remarquables par symétrie selon un axe passant par les deux autres desdits points remarquables ; et
- à déterminer un repère de calcul de la trajectoire du balayage automatique, ledit repère étant constitué par au moins deux axes, chacun comprenant une paire desdits quatre points remarquables.

Avantageusement, ledit procédé consiste à effectuer un repérage des coordonnées d'au moins un point central, ledit point central étant situé à l'intersection desdits axes.

Selon un mode de réalisation, le procédé consiste à enregistrer ledit point central au niveau du premier modèle ; et en ce que ledit recalage préliminaire s'effectue par concordance dudit point central et d'au moins un autre point remarquable.

Selon un autre mode de réalisation, il consiste à effectuer la mise en correspondance dudit premier modèle avec ledit modèle intermédiaire, par concordance desdits axes.

Selon l'application préférentielle, lesdites surfaces anatomiques correspondent au visage et que lesdits axes suivent au moins en partie l'arête nasale et une ligne frontale.

Selon une autre caractéristique facultative, le procédé consiste à déterminer un repère de référence centré sur ledit bras robotisé.

D'autres caractéristiques et avantages de l'invention ressortiront de la description détaillée qui va suivre des modes de réalisation non limitatifs de l'invention, en référence aux figures annexées dans lesquelles :
- les figures 1, 2 et 3 représentent une vue de face schématisée de trois étapes du procédé selon l'invention appliqué au visage d'un patient ;
- la figure 4 représente schématiquement une possibilité détaillée de l'étape de balayage automatique selon l'invention ; et
- les figures 5, 6 et 7 représentent schématiquement trois vues du recalage surfacique du procédé selon l'invention.

La présente invention concerne un procédé d'acquisition automatisé et assisté de surfaces anatomiques.

Plus précisément, un tel procédé combine une intervention manuelle avec une assistance robotique, puis une opération entièrement automatique robotisée.

On notera que les surfaces anatomiques au sens de l'invention peuvent comprendre toute partie du corps d'un patient. Selon l'exemple illustré sur les figures, conformément au mode de mise en oeuvre préférentiel, lesdites surfaces anatomiques correspondent au visage 1 dudit patient.

Dans un premier temps, préalablement à l'opération, est effectuée une première acquisition antérieure desdites surfaces anatomiques du patient. Une telle acquisition antérieure peut être obtenue par tout type de moyen, en particulier au travers d'un scanner ou d'un IRM.

A partir de cette acquisition antérieure, est créée une représentation en trois dimensions sous forme d'un premier modèle numérique 2.

Ensuite, en phase per opératoire, une seconde acquisition est effectuée par balayage desdites surfaces anatomiques. A partir de cette seconde acquisition est créée une représentation en trois dimensions sous forme d'un second modèle numérique 3.

Enfin, on effectue une mise en correspondance par recalage surfacique desdits premier 2 et second 3 modèles ainsi obtenus. En somme, une superposition desdites modèles 2 et 3 est réalisée de manière à faire coïncider les deux représentations, le second modèle 3 venant en recouvrement dudit premier modèle 2.

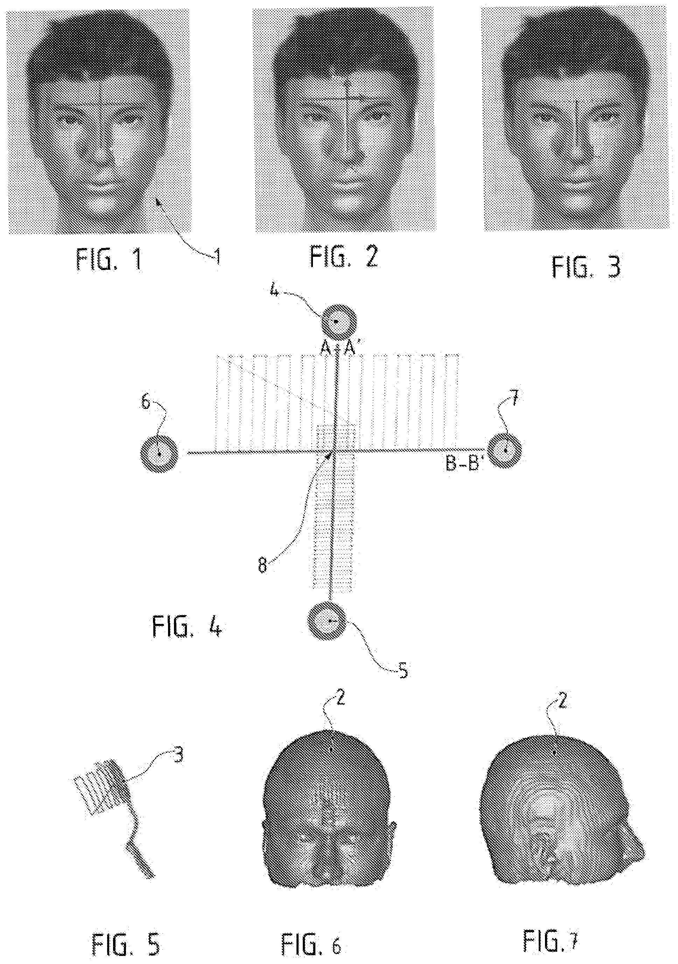

Cette étape de recalage est représentée sur les figures 5 à 7, la figure 5 représentant le second modèle 3, tandis que les figures 6 et 7 représentent la superposition dudit second modèle 3 sur le premier modèle 2, respectivement selon une vue de profil et une vue de face de la surface anatomique concernée, à savoir la tête d'un patient.

Plus particulièrement, ledit recalage peut être basé sur un algorithme de recalage de données en trois dimensions dit ICP (pour « iterative closest point »). De manière générale, l'algorithme ICP consiste à calculer de façon itérative la matrice de transformation rigide (rotation et translation) recalant le mieux deux ensembles de données, définies par leurs coordonnées dans un repère en trois dimensions.

Avantageusement, une caractéristique essentielle de la présente invention réside dans le fait que ledit balayage est réalisé par des moyens de repérage des coordonnées desdites surfaces anatomiques.

En particulier, ces moyens de repérage peuvent mesurer dans l'espace et déterminer les coordonnées de points desdites surfaces par rapport à un référentiel.

Préférentiellement, lesdits moyens de repérage sont supportés par un bras robotisé. Ce dernier est prévu mobile et asservi de manière à lui conférer des degrés de liberté de mouvement selon les trois dimensions. Dès lors, ledit référentiel, par rapport auquel sont mesurées les coordonnées des points de ladite surface anatomique, est déterminé par ledit bras robotisé.

On notera alors que le patient est immobilisé par rapport au socle sur lequel repose et se meut ledit bras robotisé.

A ce titre, ledit bras robotisé sert de repère de référence, autant au cours de l'acquisition, mais pour les autres opérations ultérieures.

En particulier, les moyens informatiques et le calculateur associés audit bras robotisé permettent de centraliser le repérage spatial du corps du patient, mais aussi des outils nécessaires pour l'acquisition et le balayage des surfaces anatomiques, comme l'émetteur positionné à l'extrémité dudit bras, mais aussi pour les outils chirurgicaux qui interviendront en phase opératoire.

Dès lors, ce repère de référence permet de recaler les informations et les coordonnées du patient, des points, mais aussi des outils d'acquisition et de chirurgie, par rapport à l'imagerie préopératoire.

En somme, le bras robotisé offre un référentiel unique permettant de repérer et coordonner dans l'espace, en temps réel, les différents éléments susmentionnés. En effet, la « chaîne modélisée » que constitue l'immobilité du corps du patient par rapport au socle du bras robotisé, ainsi que le bras lui-même jusqu'à l'extrémité de l'outil qu'il porte, est suffisante en soi, de manière à assurer un repérage de chacun des éléments qui la constitue au sein d'un repère dans l'espace. Pour obtenir un tel résultat, il est nécessaire d'initialiser une telle chaîne, notamment en repérant ses éléments constituants. Une telle opération d'initialisation peut s'effectuer préalablement à l'acquisition et tout au long de celle-ci, mais aussi postérieurement et en cours de l'intervention, au travers d'étapes de mise à jour de ladite chaîne. Ces mises à jour interviennent automatiquement en fonction du positionnement des outils et élément utilisés, solidaires dudit bras robotisé.

Dès lors, un caractère répétable intervient dans le positionnement et le déplacement des outils et éléments, alors que cet aspect reproductif était inenvisageable ou impossible lors du travail entièrement manuel d'un praticien, les rendant opérateur dépendant.

Par ailleurs, il est également possible de rendre indépendant le corps du patient du bras robotisé et de son socle. Dès lors, il convient d'opérer une acquisition en temps réel de la position dudit patient, afin d'en connaître avec exactitude les changements pour s'y adapter.

Enfin, l'extrémité du bras robotisé peut aussi porter simultanément des moyens nécessaires pour l'acquisition et des outils pour l'intervention. En effet, il est alors possible d'envisager, au travers d'éléments et d'outils miniaturisés, d'intégrer des technologies de localisation, comme un laser, des ultrasons, une caméra, des outils mécaniques ou des éléments de télémétrie de surface ou percutanée, couplées à des outillages, notamment chirurgicaux. Il est alors possible de connaître en temps réel le positionnement et le déplacement de l'un ou l'autre de ces éléments, permettant une automatisation des mouvements et trajectoires de chacun d'entre eux, combinée à des acquisitions et recalages surfaciques en trois dimensions.

Selon un mode de réalisation, lesdits moyens de repérage peuvent être prévus sans contact, notamment sous la forme d'un émetteur de rayonnement, par exemple lumineux, tel un faisceau laser, couplé à un capteur de distance. Plus particulièrement, lesdits moyens de repérage peuvent se présenter sous la forme d'un télémètre laser.

D'autres émetteurs peuvent être envisagés, utilisant des faisceaux optiques, des ondes acoustiques comme les ultrasons ou bien des ondes radio.

Ainsi, de tels moyens de repérage, situés à l'extrémité mobile dudit bras robotisé, peuvent se déplacer autour des surfaces anatomiques à balayer.

A ce titre, une caractéristique essentielle de l'invention réside dans le fait que le balayage est divisé est deux étapes successives.

Une première étape du balayage consiste à effectuer un repérage préliminaire des coordonnées de points remarquables desdites surfaces anatomiques par déplacement manuel, assisté par ledit bras robotisé, desdits moyens de repérage au niveau desdits points remarquables. Ce repérage préliminaire permet de déterminer une zone de balayage desdites surfaces anatomiques.

En somme, le praticien pilote lui-même le déplacement des moyens de repérage, toujours solidaires dudit bras robotisé, pour les positionner et mesurer les coordonnées de points spécifiques des surfaces anatomiques.

Cette étape est opérateur dépendant, mais suffisamment simple à mettre en oeuvre pour assurer une reproductibilité et une précision satisfaisante des coordonnées desdits points remarquables.

La seconde étape consiste à effectuer un balayage automatique de la zone déterminée par lesdits points remarquables, au travers du bras robotisé seul.

Ainsi, le repérage manuel permet d'améliorer le balayage automatique en bornant et délimitant la zone au sein de laquelle les coordonnées seront mesurées, augmentant la précision, limitant les risques d'extrapolation et offrant une capacité d'adaptation de l'invention aux différentes morphologie des surfaces anatomiques.

Selon un mode particulier de réalisation, ledit repérage manuel enregistre les coordonnées d'au moins trois points remarquables parmi 4, 5, 6 ou 7 pour construire un repère.. Un quatrième point peut être déterminé par symétrie par rapport aux trois autres points déjà ciblés. En particulier, dans le cas d'un visage, ledit quatrième point peut être obtenu à partir d'un desdits trois points remarquables par symétrie par rapport à un axe passant par les deux autres desdits points remarquables. D'autres points intermédiaires remarquables peuvent être repérés manuellement par le praticien, en fonction des cas et de la morphologie des surfaces anatomiques concernées.

Puis, on détermine un repère de calcul de la trajectoire du balayage automatique, ledit repère étant constitué par au moins deux axes A-A' et B-B', chacun comprenant une paire 4,5 et 6,7 desdits quatre points remarquables.

Dans l'exemple de mise en oeuvre représenté sur les figures 1 à 3, lesdites surfaces anatomiques correspondent au visage 1. De plus, lesdits axes A-A' et B-B' suivent respectivement au moins en partie l'arête nasale et une ligne frontale, l'arête nasale étant sensiblement verticale tandis que la ligne frontale est sensiblement horizontale. Lesdits points remarquables peuvent alors correspondre : pour le point 4 au milieu du front, pour le point 5 à l'extrémité de l'os nasal, pour le point 6 à un point de l'extrémité gauche du front tandis que le point 7 correspond à l'extrémité opposée.

On notera que les points 4 et 5 déterminent la hauteur de la zone à balayer, tandis que les points 6 et 7, situés de chaque côté du visage 1, permettent de déterminer la largeur de ladite zone.

De plus, l'invention prévoit d'effectuer une première mise en correspondance mise en correspondance à l'aide dudit repère ainsi défini.

Pour ce faire, l'invention consiste à effectuer un repérage des coordonnées d'au moins un point central 8. Tout particulièrement, ledit point central 8 est situé à l'intersection desdits axes A-A' et B-B'.

Ledit point central 8 servira de centre afin d'effectuer ce recalage surfacique préliminaire.

Pour y parvenir, il convient d'enregistrer ledit point central 8 au niveau du premier modèle 2, à savoir sur l'imagerie réalisée avant l'opération.

Puis, on crée une représentation intermédiaire en trois dimensions sous forme d'un modèle intermédiaire numérique. En somme, ce modèle intermédiaire peut inclure l'un et/ou l'autre des points remarquables 4 à 7, ainsi que le point central O et/ou les axes A-A' et B-B'.

Ensuite, est effectuée une mise en correspondance, par recalage préliminaire dudit premier modèle 2 avec ledit modèle intermédiaire, par concordance dudit point central 8 et d'au moins un autre point remarquable 4 à 7. Cette mise en correspondance intermédiaire peut aussi s'effectuer à l'aide dudit repère ainsi défini, par concordance desdits axes A-A' et B-B'.

Ce pré recalage permet ainsi d'ajuster plus efficacement les modèles 1 et 2 lors du recalage final.

Ledit recalage préliminaire (et le temps de calcul informatique associé) peut aussi intervenir pendant l'étape de balayage automatique.

A ce titre, une fois la zone de balayage déterminée, la trajectoire de balayage est calculée afin d'optimiser le nombre et la répartition des points repérés au sein de ladite zone.

Un exemple non limitatif de trajectoire est schématisé sur la figure 4. On constate que la trajectoire suit l'axe B-B' du point 6 vers le point 7, en partie haute du repère, puis vient suivre symétriquement l'axe A-A' du haut vers le bas, depuis le dessous du point 4 jusqu'au point 5.

On notera que dans l'exemple, le déplacement s'effectue sous forme d'une trajectoire crénelée et continue. Toutefois, tout type de déplacement peut être envisagé, selon différentes courbes, continues ou discontinues, adaptées en fonction de la morphologie de la zone anatomique concernée.

Par ailleurs, toutes les données numériques au sens de la présente invention (modèles, coordonnées ou algorithme) et leur mise en oeuvre (enregistrement, modification ou visualisation) s'opèrent au travers de moyens informatiques adaptés. Il en va de même pour la programmation et les commandes numériques dudit bras robotisé.

Le procédé d'acquisition sans contact selon l'invention offre une méthodologie fiable et reproductible, rapide et facile à mettre en oeuvre pour tout praticien, pour un résultat précis, s'affranchissant des erreurs liées à l'opérateur tout en conservant une amélioration du fait de l'adaptabilité en fonction du praticien et de son expérience médicale. En particulier, l'opérateur peut corriger la trajectoire initiale, conférant un aspect intelligent et coopératif, mais aussi de la flexibilité aux avantages apportés par la rigueur et la précision de l'automatisme robotique.

Bien entendu, l'invention n'est pas limitée aux exemples illustrés et décrits précédemment qui peuvent présenter des variantes et modifications sans pour autant sortir du cadre de l'invention.

## Revendications

1. Procédé d'acquisition automatisé et assisté de surfaces anatomiques lors de la préparation d'une intervention chirurgicale, **caractérisé en ce qu'**il comporte :
- une étape d'acquisition préopératoire des surfaces anatomiques de manière à créer un premier modèle numérique (2) ;
- une étape d'acquisition manuelle peropératoire au cours de laquelle un opérateur manipule un bras robotisé équipé de moyens de repérage des surfaces anatomiques de façon à réaliser un repérage préliminaire des coordonnées de points remarquables (4, 5, 6, 7) ;
- une étape de construction d'un repère en utilisant des coordonnées des points remarquables (4, 5, 6, 7) ;
- une étape d'acquisition automatisée par balayage automatique d'une zone de balayage, définie par le repère, des surfaces anatomiques, l'acquisition automatisée assurant la création d'un second modèle numérique (3) ;
ledit balayage étant réalisé par les moyens de repérage des coordonnées desdites surfaces anatomiques;
- une étape d'utilisation des coordonnées des points remarquables (4, 5, 6, 7) et du repère de manière à créer un modèle numérique intermédiaire ;
- une étape de recalage préliminaire du premier modèle numérique (2) avec le modèle numérique intermédiaire ; et
- une étape de mise en correspondance par recalage entre le premier modèle numérique (2) préalablement recalé et le second modèle numérique (3) de façon à former un modèle numérique final.

2. Procédé d'acquisition selon la revendication 1, **caractérisé en ce que** l'étape d'acquisition manuelle comporte :
- un repérage des coordonnées d'au moins trois points remarquables (4, 5, 6, 7) ; et
- un des trois points remarquables (4, 5, 6, 7) permettant de déterminer, un quatrième point remarquable (4, 5, 6, 7), par symétrie selon un axe passant par les deux autres points remarquables (4, 5, 6, 7).

3. Procédé d'acquisition selon la revendication 2, **caractérisé en ce que** lors de l'étape de construction du repère, chacun des deux axes (A-A', B-B') définissant le repère est déterminé par deux des quatre points remarquables (4, 5, 6, 7).

4. Procédé d'acquisition selon la revendication 3, **caractérisé en ce que** lors de l'acquisition automatisée le bras robotisé se déplace selon les deux axes (A-A', B-B') en décrivant un mouvement transversal continu.

5. Procédé d'acquisition selon la revendication 3, **caractérisé en ce que** l'étape de recalage préliminaire consiste à effectuer la mise en correspondance du premier modèle numérique (2) avec le modèle numérique intermédiaire, par correspondance des deux axes (A-A', B-B').

6. Procédé d'acquisition selon la revendication 3, **caractérisé en ce que** l'intersection des deux axes (A-A', B-B') définit des coordonnées d'un point central (8).

7. Procédé d'acquisition selon la revendication 6, **caractérisé en ce que** l'étape de recalage préliminaire consiste à enregistrer le point central (8) du repère au niveau du première modèle numérique (2), le recalage préliminaire s'effectuant par concordance du point central (8) et d'au moins un autre point remarquable (4, 5, 6, 7).

8. Procédé d'acquisition selon la revendication 3, **caractérisé en ce que** les surfaces anatomiques correspondent au visage (1), les axes (A-A', B-B') suivant au moins en partie l'arête nasale et une ligne frontale.

9. Procédé d'acquisition selon la revendication 4, **caractérisé en ce que** le bras se déplace sous forme d'une trajectoire crénelée et continue.

10. Procédé d'acquisition selon l'une des revendications 1 à 9, **caractérisé en ce que** lors de l'étape d'acquisition automatisée le repère définit une zone de balayage dont la hauteur et la largeur sont définis par les points remarquables opposés (4, 5, 6, 7).

11. Procédé d'acquisition selon l'une des revendications 1 à 10, **caractérisé en ce qu'**un repère de référence est centré sur le bras robotisé.

## Patentansprüche

1. Automatisiertes und unterstütztes Erfassungsverfahren von anatomischen Flächen bei der Vorbereitung eines chirurgischen Eingriffs, **dadurch gekennzeichnet, dass** es folgendes umfasst:
- einen präoperativen Erfassungsschritt von anatomischen Flächen, um ein erstes digitales Modell (2) zu erstellen;
- einen präoperativen manuellen Erfassungsschritt, in dessen Verlauf ein Bediener einen Roboterarm handhabt, der mit Markierungsmitteln von anatomischen Flächen ausgestattet ist, um eine vorbereitende Markierung der Koordinaten von bedeutenden Punkten (4, 5, 6, 7) durchzuführen;
- einen Aufbauschritt eines Markierungspunkts unter Verwendung der Koordinaten der bedeutenden Punkte (4, 5, 6, 7);
- einen automatisierten Erfassungsschritt durch automatische Abtastung einer Abtastzone, die durch den Markierungspunkt der anatomischen Flächen definiert ist, wobei die automatisierte Erfassung die Erstellung eines zweiten digitalen Modells (3) gewährleistet; wobei die Abtastung von den Markierungsmitteln der Koordinaten dieser anatomischen Flächen durchgeführt wird;
- einen Verwendungsschritt der Koordinaten der bedeutenden Punkte (4, 5, 6, 7) und des Markierungspunkts, um ein digitales Zwischenmodell zu erstellen;
- einen vorbereitenden Korrekturschritt des ersten digitalen Modells (2) mit dem digitalen Zwischenmodell; und
- einen Abgleichschritt durch Korrektur zwischen dem ersten digitalen Modell (2), das vorher korrigiert wurde, und dem zweiten digitalen Modell (3), um endgültiges digitales Modell zu bilden.

2. Erfassungsverfahren nach Anspruch 1, **dadurch gekennzeichnet, dass** der manuelle Erfassungsschritt folgendes umfasst:
- eine Markierung der Koordinaten aus mindestens drei bedeutenden Punkten (4, 5, 6, 7); und
- wobei einer der drei bedeutenden Punkte (4, 5, 6, 7) die Bestimmung eines vierten bedeutenden Punkts ermöglicht (4, 5, 6, 7), durch Symmetrie gemäß einer Achse, die durch die zwei anderen bedeutenden Punkte (4, 5, 6, 7) verläuft.

3. Erfassungsverfahren nach Anspruch 2, **dadurch gekennzeichnet, dass** bei dem Aufbauschritt des Markierungspunkts jede der zwei Achsen (A-A', B-B'), die den Markierungspunkt definieren, durch zwei von vier bedeutenden Punkten (4, 5, 6, 7) bestimmt ist.

4. Erfassungsverfahren nach Anspruch 3, **dadurch gekennzeichnet, dass** sich der Roboterarm bei der automatisierten Erfassung entlang von zwei Achsen (A-A', B-B') bewegt und eine kontinuierliche transversale Bewegung beschreibt.

5. Erfassungsverfahren nach Anspruch 3, **dadurch gekennzeichnet, dass** der vorbereitende Korrekturschritt darin besteht, den Abgleich des ersten digitalen Modells (2) mit dem digitalen Zwischenmodell durch Entsprechung der zwei Achsen (A-A', B-B') durchzuführen.

6. Erfassungsverfahren nach Anspruch 3, **dadurch gekennzeichnet, dass** der Schnittpunkt der zwei Achsen (A-A', B-B') die Koordinaten eines Mittelpunkts (8) definiert.

7. Erfassungsverfahren nach Anspruch 6, **dadurch gekennzeichnet, dass** der vorbereitende Korrekturschritt darin besteht, den Mittelpunkt (8) des Markierungspunkts auf Ebene des ersten digitalen Modells (2) zu registrieren, wobei die vorbereitende Korrektur durch Entsprechung des Mittelpunkts (8) und mindestens einem anderen bedeutenden Punkt (4, 5, 6, 7) ausgeführt wird.

8. Erfassungsverfahren nach Anspruch 3, **dadurch gekennzeichnet, dass** die anatomischen Flächen dem Gesicht (1) entsprechen, wobei die Achsen (A-A', B-B') mindestens teilweise dem Nasenrücken und einer frontalen Linie folgen.

9. Erfassungsverfahren nach Anspruch 4, **dadurch gekennzeichnet, dass** sich der Arm in Form einer gezackten und kontinuierlichen Bahn bewegt.

10. Erfassungsverfahren nach einem der Ansprüche 1 bis 9, **dadurch gekennzeichnet, dass** bei dem automatisierten Erfassungsschritt der Markierungspunkt eine Abtastzone definiert, deren Höhe und Breite durch die gegenüberliegenden bedeutenden Punkte (4, 5, 6, 7) definiert sind.

11. Erfassungsverfahren nach einem der Ansprüche 1 bis 10, **dadurch gekennzeichnet, dass** nur eine Bezugsmarkierung auf dem Roboterarm zentriert ist.

## Claims

1. Method for the automated and assisted acquisition of anatomical surfaces when preparing for a surgical operation, **characterised in that** it comprises:
- A preoperative stage to acquire anatomical surfaces so that a first numerical model (2) is created;
- A manual preoperative acquisition stage during which an operator manipulates a robotic arm fitted with means of registering anatomical surfaces in such a way that a preliminary registration of the co-ordinates of significant points (4, 5, 6, 7) is obtained;
- a stage for constructing a reference point using the co-ordinates of the significant points (4, 5, 6, 7);
- an automated acquisition stage to automatically scan a scanning zone, defined by the reference point, of the anatomical surfaces, wherein the automated acquisition provides for the creation of a second numerical model (3); wherein said scanning is performed by the means of registering the co-ordinates of the said anatomical surfaces;
- a stage utilising the co-ordinates of the significant points (4, 5, 6, 7) and of the reference point so that an intermediate numerical model is created;
- a stage for the preliminary adjustment of the first numerical model (2) with the intermediate numerical model; and
- A stage to form a match for adjustment between the first numerical model (2), previously adjusted, and the second numerical model (3) in such a way that a final numerical model is formed.

2. Acquisition method according to claim 1, **characterised in that** the manual acquisition stage comprises:
- registration of the co-ordinates of at least three significant points (4, 5, 6, 7); and
- one of the three significant points (4, 5, 6, 7) providing for the determination of a fourth significant point (4, 5, 6, 7) by symmetry according to an axis passing through the two other significant points (4, 5, 6, 7).

3. Acquisition method according to claim 2, **characterised in that**, at the stage where the reference point is constructed, each of the two axes (A-A', B-B') defining the reference point is determined by two of the four significant points (4, 5, 6, 7).

4. Acquisition method according to claim 3, **characterised in that**, during the automated acquisition, the robotic arm is moved in accordance with the two axes (A-A', B-B') describing a continuous, transverse movement.

5. Acquisition method according to claim 3, **characterised in that** the preliminary adjustment stage consists of matching the first numerical model (2) and the intermediate numerical model by aligning the two axes (A-A', B-B').

6. Acquisition method according to claim 3, **characterised in that** the intersection of the two axes (A-A', B-B') defines the co-ordinates of a central point (8).

7. Acquisition method according to claim 6, **characterised in that** the preliminary adjustment stage consists of entering the central point (8) of the reference point in the first numerical model (2), wherein the preliminary adjustment is performed by correlating the central point (8) with at least one other significant point (4, 5, 6, 7).

8. Acquisition method according to claim 3, **characterised in that** the anatomical surfaces correspond to the face (1), wherein the axes (A-A', B-B') follow at least partially the nasal crest and a hairline.

9. Acquisition method according to claim 4, **characterised in that** the arm is moved in the shape of a continuous, crenulated path.

10. Acquisition method according to any one of claims 1 to 9, **characterised in that**, as the automated acquisition stage is being performed, the reference point defines a scanning zone, the height and width of which are defined by the opposing significant points (4, 5, 6, 7).

11. Acquisition method according to any one of claims 1 to 10, **characterised in that** a reference point is centred on the robotic arm.
